# EUROPEAN PATENT APPLICATION

(11) **EP 4 148 107 A1**
(43) Date of publication of application: **15.03.2023**
(21) Application number: 22194998.5
(22) Date of filing: 12.09.2022
(51) Int. Cl.: C10G 65/04, C10G 69/06

(54) **PROCESS FOR DEISOMERIZING LIGHT PARAFFINS**

(30) Priority: 13.09.2021 US 202163243407 P
(71) Applicant: UOP LLC, Des Plaines, Illinois 60017-5017 (US)
(72) Inventor: ROKKAM, Ram Ganesh, Charlotte, 28202 (US); PLOENTHAM, Cora Wang, Charlotte, 28202 (US); FUNK, Gregory A., Charlotte, 28202 (US); JANI, Priyesh Jayendrakumar, Charlotte, 28202 (US)
(74) Representative: Boult Wade Tennant LLP

(57) **Abstract**

A process for increasing conversion and selectivity to normal paraffins is achieved by reducing the hydrogen to hydrocarbon ratio for paraffin feeds with substantial butanes. A separator may be used to remove excess hydrogen from a first isomerate before a second isomerization step that may isomerize additional butanes perhaps generated in the first isomerization step.

## Description

### CROSS-REFERENCE TO RELATED APPLICATIONS

This application claims priority from United States Provisional Application No. 63/243,407, filed September 13, 2022, which is incorporated herein in its entirety.

### FIELD

The field is processes for isomerizing light paraffins to increase the concentration of normal paraffins.

### BACKGROUND

Ethylene and propylene are important chemicals for use in the production of other useful materials, such as polyethylene and polypropylene. Polyethylene and polypropylene are two of the most common plastics found in use today and have a wide variety of uses. Uses for ethylene and propylene include the production of vinyl chloride, ethylene oxide, ethylbenzene and alcohol.

The great bulk of the ethylene consumed in the production of the plastics and petrochemicals such as polyethylene is produced by the thermal cracking of higher molecular weight hydrocarbons. Steam is usually mixed with the feed stream to the cracking furnace to reduce the hydrocarbon partial pressure and enhance olefin yield and to reduce the formation and deposition of carbonaceous material in the cracking reactors. The process is therefore often referred to a steam cracking or pyrolysis.

The composition of the feed to the steam cracking reactor affects the product distribution. The propensity of particular hydrocarbons to crack is greater than others. The tendency of the hydrocarbons to crack to ethylene normally ranks in the following order: normal paraffins; iso-paraffins; olefins; naphthenes; and aromatics.

One way to upgrade light naphtha is first to separate the naphtha into a normal paraffin rich stream and a non-normal paraffin rich stream; and subsequently convert a substantial amount of the non-normal paraffin stream in an isomerization zone in the presence of a catalyst into normal paraffins. Isomerization can produce normal butanes with the other normal paraffins which must be managed. Separating isoparaffins intended for further isomerization from normal paraffins intended for steam cracking requires a series of fractionation columns and can substantially increase capital cost.

An efficient process for separating and converting the iso-paraffins in light naphtha to normal paraffins would significantly increase the profitability of steam cracking operations by increasing the yield of high value ethylene and propylene.

### BRIEF SUMMARY

A process and apparatus for isomerizing isoparaffins to normal paraffins comprises isomerizing a first paraffin stream to provide a first isomerate stream, separating the first isomerate stream into a first vapor isomerate stream and a first liquid isomerate stream, and isomerizing said first liquid isomerate stream to provide a second isomerate stream. The process and apparatus separate isomerization of C4 paraffins from C5 paraffins because advantageous conditions are different for each isomerization reactant.

Additional details and embodiments of the disclosure will become apparent from the following detailed description of the disclosure.

### BRIEF DESCRIPTION OF THE DRAWINGS

FIG. 1 is a schematic view of a process of the present disclosure;
FIG. 2 is a schematic view of an alternate process of FIG. 1;
FIG. 3 is a schematic view of a further alternate process of FIG. 1;
FIG. 4 is a schematic view of an alternate process of FIG. 2;
FIG. 5 is a schematic view of an alternate process of FIG. 4;
FIG. 6 is a schematic view of an alternate process of FIG. 5; and
FIG. 7 is a schematic view of an alternate process of FIG. 6.

### DEFINITIONS

The term "communication" means that fluid flow is operatively permitted between enumerated components, which may be characterized as "fluid communication".

The term "downstream communication" means that at least a portion of fluid flowing to the subject in downstream communication may operatively flow from the object with which it fluidly communicates.

The term "upstream communication" means that at least a portion of the fluid flowing from the subject in upstream communication may operatively flow to the object with which it fluidly communicates.

The term "direct communication" means that fluid flow from the upstream component enters the downstream component without passing through any other intervening vessel.

The term "bypass" means that the object is out of downstream communication with a bypassing subject at least to the extent of bypassing.

The term "column" means a distillation column or columns for separating one or more components of different volatilities. Unless otherwise indicated, each column includes a condenser on an overhead of the column to condense and reflux a portion of an overhead stream back to the top of the column and a reboiler at a bottom of the column to vaporize and send a portion of a bottoms stream back to the bottom of the column. Feeds to the columns may be preheated. The top pressure is the pressure of the overhead vapor at the vapor outlet of the column. The bottom temperature is the liquid bottom outlet temperature. Overhead lines and bottoms lines refer to the net lines from the column downstream of any reflux or reboil to the column. Stripper columns may omit a reboiler at a bottom of the column and instead provide heating requirements and separation impetus from a fluidized inert media such as steam. Stripping columns typically feed a top tray and take main product from the bottom.

As used herein, the term "a component-rich stream" or "a stream rich in a component" means that the rich stream coming out of a vessel has a greater concentration of the component than any other stream from the vessel.

As used herein, the term "a component-lean stream" or "a stream lean in a component" means that the lean stream coming out of a vessel has a smaller concentration of the component than any other stream from the vessel.

The term "Cx" is to be understood to refer to molecules having the number of carbon atoms represented by the subscript "x". Similarly, the term "Cx-" refers to molecules that contain less than or equal to x and preferably x and less carbon atoms. The term "Cx+" refers to molecules with more than or equal to x and preferably x and more carbon atoms.

As used herein, the term "separator" means a vessel which has an inlet and at least an overhead vapor outlet and a bottoms liquid outlet and may also have an aqueous stream outlet from a boot. A flash drum is a type of separator which may be in downstream communication with a separator that may be operated at higher pressure.

As used herein, the term "predominant" or "predominate" means greater than 50%, suitably greater than 75% and preferably greater than 90%.

As used herein, the term "T5" or "T95" means the temperature at which 5 mass percent or 95 mass percent, as the case may be, respectively, of the sample boils using ASTM D-86 or TBP.

As used herein, the term "initial boiling point" (IBP) means the temperature at which the sample begins to boil using ASTM D-7169, ASTM D-86 or TBP, as the case may be.

As used herein, the term "end point" (EP) means the temperature at which the sample has all boiled off using ASTM D-7169, ASTM D-86 or TBP, as the case may be.

### DETAILED DESCRIPTION

In a process to increase the concentration of normal paraffins in a feed stream, C5+ isoparaffins are isomerized to C5+ normal paraffins. However, we have found that isomerization of C5+ paraffins generates isobutanes. Furthermore, we found that isobutanes exhibit low conversion in a C5 isomerization reactor and do not reach the applicable normal C4/C4 equilibrium ratio because the presence of heavies and aromatic rings in the C5+ paraffin feed and high hydrogen partial pressure inhibit C4 conversion.

With these findings we propose to use a separator between the C5 isomerization reactor and the C4 isomerization reactor to separate out hydrogen so as to enable sending only minimal hydrogen sufficient to provide necessary and perhaps stochiometric quantities to the C4 isomerization unit. If the non-normal paraffin feed stream comprises substantial C4 paraffins, the C4 paraffins are first separated and converted in a C4 isomerization unit while the C5+ paraffins are converted in a C5+ isomerization unit.

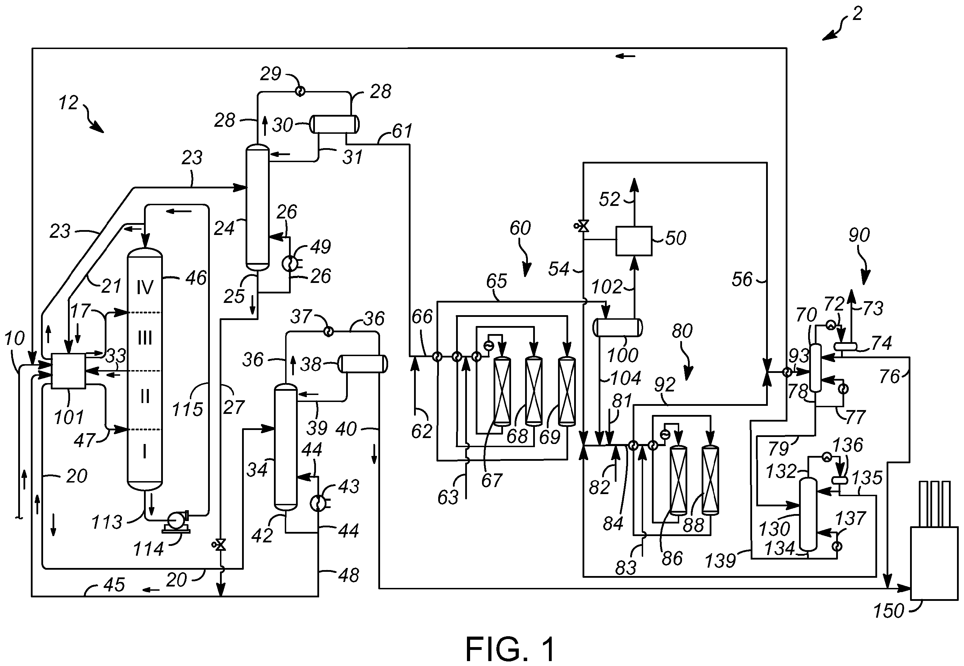

In the process and apparatus 2 in FIG. 1, a naphtha feed stream in line 10 is preferably a hydrotreated light naphtha stream comprising substantially C4 to C6 hydrocarbons having a T90 between about 40°C and about 90°C. The end point is taken to minimize the presence of hydrocarbons with more than six carbon atoms in the feed. Suitably no more than about 30 wt% C7+ hydrocarbons, preferably no more than about 20 wt% C7+ hydrocarbons and more preferably no more than about 10 wt% C7+ hydrocarbons can be present in the light naphtha feed stream. The naphtha feed stream may comprise normal paraffins, iso-paraffins, naphthenes, and aromatics.

Normal paraffins yield more light olefins in a steam cracking unit. Hence, it is desired to increase the concentration of normal paraffins in the feed stream 10. The first step in the process is a step of separating the naphtha feed stream into a normal paraffin-rich stream and a non-normal paraffin-rich stream. Normal molecules are defined to mean straight chain molecules such as normal butane, normal hexane, and normal pentane. The most efficient process for such a separation utilizes adsorption. In an aspect, an adsorption separation unit 12 is used to separate normal paraffins from non-normal paraffins.

The naphtha feed stream is delivered to the process in a feed line 10 and passed to the adsorption separation unit 12. The feed stream in feed line 10 is passed through a valve 101 in the adsorption separation unit 12 which delivers the feed to an appropriate bed in an adsorbent vessel 46.

The feed stream in feed line 10 is separated into a normal paraffins stream and a non-normal paraffins stream. Normal paraffins of the naphtha mixture selectively enter or occlude into the porous structure of the adsorbent components but branched or cyclic non-normal chain paraffins do not typically enter the pores. The non-normal paraffins exit the process as a raffinate stream. In an aspect, the normal butanes enter or occlude into the porous structure of the adsorbent components while the isobutanes do not typically enter the pores. The same dynamic occurs for the C5-C7 paraffins. In an aspect, the normal C5-C7 paraffins enter or occlude into the porous structure of the adsorbent while the iso-C5-C7 paraffins do not typically enter the pores. Consequently, the butanes are separated in the adsorption separation unit 12 like the C5-C7 hydrocarbons.

To provide a useful method for separation of normal from non-normal paraffins, it is necessary to desorb the occluded normal paraffins. In the disclosed process, normal nonane or normal decane or even heavier normal paraffin can suitably be used as a desorbent to desorb normal paraffins in an extract-desorbent stream.

The adsorbent used in the adsorption vessel preferably comprises aluminosilicate molecular sieves having relatively uniform pore diameters of about 5 Angstroms. The preferred adsorbent is provided by commercially available type 5A molecular sieves produced and sold by UOP LLC in Des Plaines, Illinois.

The adsorbent vessel 46 may comprise a series of vertically spaced, separate beds interconnected by a pipe 115 between the bottom of one bed and the top of its downstream adjacent bed. The valve 101 may comprise a manifold arrangement or a rotary valve for advancing the points of inlet and outlet of respective streams in a downstream direction. The adsorbent vessel 46 operates in a downflow mode, although upflow may be suitable. The adsorbent vessel 46 is shown to have four main zones I-IV for simplicity, though these zones may be further subdivided when accounting for different flushing schemes. The overall process may have other numbers of beds, such as eight, twelve or twenty-four beds, divided among the four main zones I-IV.

The feed stream is introduced through feed line 10 through valve 101 which is positioned to send the feed stream through line 47 into the adsorbent vessel 46 between Zones I and II. The extract is withdrawn between Zones II and III in line 33, transported through the valve 101 in an extract line 20 to an extract fractionation column 34 to separate desorbent from extract. The desorbent is introduced through desorbent line 45 through the valve 101 which is positioned to send the desorbent through a desorbent line 17 into the process between Zones III and IV. The raffinate is withdrawn between Zones IV and I through a raffinate line 21, through the valve 101 and through line 23 to the raffinate fractionation column 24.

Simulated countercurrent flow is achieved by periodically advancing downstream the introduction point of the feed stream and the desorbent stream while simultaneously and equally advancing downstream the withdrawal point of the raffinate stream and the extract stream. The Zone I is defined as the zone bounded between the feed stream inlet and the raffinate outlet; the Zone II is defined as the zone bounded between the extract stream outlet and the desorbent inlet; the Zone III is defined as the zone bounded between the desorbent inlet and the extract outlet; and the Zone IV is defined as the zone bounded between the raffinate stream outlet and the desorbent stream inlet. Typical liquid phase operation is preferred, for example, at temperatures from about 50°C to about 300°C, and more particularly no more than about 260°C, and pressures from slightly super atmospheric to about 30 atmospheres.

Raffinate, characterized as less adsorbed in the adsorption vessel, is withdrawn from the adsorption vessel 46 in the raffinate line 21 through the valve 101 and enters the raffinate fractionation column 24 through line 23. The raffinate stream in line 23 can be considered a paraffin stream or an isoparaffin stream or a paraffin fractionation stream. Since it is desired to obtain a normal paraffin product, the raffinate fractionation column 24 is operated to separate two fractions, a raffinate overhead stream rich in non-normal paraffins, in an embodiment, rich in C7- non-normal paraffins also rich in C5- isoparaffins so it can be characterized as an isopentane stream or a high isoparaffin stream, and a desorbent bottoms stream rich in normal paraffin desorbent, in an embodiment, rich in C9+ normal paraffins. The raffinate overhead stream is withdrawn from the raffinate fractionation column 24 in an overhead line 28, condensed in a cooler 29 and fed to a separator 30. A portion of the condensed raffinate overhead is recycled to the raffinate fractionation column 24 as reflux through a reflux line 31 and the remaining portion of the condensed raffinate overhead is withdrawn through a net raffinate overhead line 61. The raffinate overhead stream is rich in C5- isoparaffins which can be transported to the first isomerization unit 60. In this embodiment, the raffinate stream in line 23 comprises no more than about 5 to about 10 wt% butanes.

The raffinate bottoms stream is withdrawn from the raffinate fractionation column 24 through a bottoms line 25 where a portion of the raffinate bottoms stream flows through a reboiler line 26, reboiler heater 49 and returns heated to the raffinate fractionation column 24. The remaining portion of said raffinate bottoms stream flows through a net bottoms line 27 as a normal paraffin rich stream, particularly rich in normal C9+ paraffins. The raffinate bottoms stream comprising a raffinate desorbent stream in line 27 can be recycled to the adsorption vessel 46 in the desorbent line 45 perhaps after joining an extract bottoms stream in line 48. The raffinate fractionation column 24 operates in a bottoms temperature range of about 250 to about 290°C and an overhead pressure of about 450 to about 550 kPa (gauge).

Extract is more or selectively adsorbed on the adsorbent in the adsorption vessel 46. The desorbent displaces the selectively adsorbed normal paraffins from the solid adsorbent in desorbent bed III of adsorbent vessel 46. The extract and desorbent are withdrawn in line 33, and the valve 101 connects line 33 with line 20. Extract and desorbent withdrawn from the adsorption vessel in the extract line 33 connected through the valve 101 is directed in line 20 to the extract fractionation column 34. Since it is desired to obtain a normal paraffin product, the extract fractionation column 34 is operated to separate two fractions, an extract overhead stream rich in normal paraffins, in an embodiment, rich in C4-C7 normal paraffins, and a desorbent bottoms stream rich in normal paraffin desorbent, in an embodiment, rich in C9+ normal paraffins. The extract overhead stream is withdrawn from the extract fractionation column 34 in an overhead line 36, condensed in a cooler 37 and fed to a separator 38. A portion of the condensed extract overhead is recycled to the extract fractionation column 34 as reflux through a reflux line 39 and the remaining portion of the condensed extract overhead is withdrawn through a net extract overhead line 40. The extract overhead stream is rich in C4-C7 normal paraffins which can be recovered or taken as steam cracking unit feed and fed to the steam cracking unit 150 in line 40.

The extract bottoms stream is withdrawn from extract fractionation column 34 through a bottoms line 42 where a portion of the extract bottoms stream flows through a reboiler line 44, reboiler heater 43 and returns heated to the extract fractionation column 34. A remaining portion of the extract bottoms stream flows through line 48 as a normal paraffin rich stream, particularly rich in normal C9+ paraffins. The extract bottoms stream in line 48 comprising an extract desorbent stream can join the raffinate bottoms stream in line 27 comprising a raffinate desorbent stream. Both can be recycled in the desorbent line 45 through the valve 101 to the adsorption vessel 46 in the desorbent line 47. The extract fractionation column 34 operates in bottoms temperature range of about 225 to about 275°C and an overhead pressure of about 250 to about 350 kPa (gauge).

The net raffinate overhead stream in the net raffinate overhead line 61 characterized as a first paraffin stream should be isopentane rich and comprise less than about 5 to about 10 mol% butanes. The first paraffin stream comprising an isopentane stream in the net raffinate overhead line 61 may be combined with a first hydrogen stream in a first hydrogen line 62 and heated by heat exchange with a first isomerate stream and fed to a first isomerization unit 60 in a first isomerization feed stream in a first isomerization feed line 66. The first isomerization unit 60 may be operated under high isomerization conditions because higher paraffins are in the first isomerization feed stream. In the first isomerization unit 60, isopentane and/or isohexane and perhaps isoheptane, in the presence of hydrogen provided by the first hydrogen line 62 and a high isomerization catalyst, are converted to increase the concentration of normal paraffins: ethane, propane, normal butane, normal pentane and normal hexane. Reactions that promote the production of normal paraffins include isoparaffin disproportionation reactions, ring saturation, opening of aromatics and cyclics, reverse isomerization of iso-paraffins, and paraffin hydrocracking reactions.

Cracking of some of the paraffins can occur in the first isomerization unit 60 to produce C4- paraffins. It is believed that the paraffin disproportionation reactions occur by the combination of two iso-paraffins followed by scission into one lighter paraffin and one heavier paraffin. For example, two isopentanes can combine and form an isobutane and an isohexane in the presence of hydrogen. The isobutanes can further react via disproportionation to form a propanes and isopentanes. A portion of the produced isobutanes also converts to normal butanes via isomerization reactions. Production of normal propane and butane via disproportionation and isomerization reactions occurs with low production of low-value undesired methane as a cracked product. Thus, there is an increase in the overall yield of the normal paraffins in the first isomerization unit 60.

In the first isomerization reactors 67-69, hydrocracking of the isopentane and/or isohexane occurs to produce methane, ethane, propane, and isobutane. The isobutane can further react via disproportionation reactions and/or isomerization to further produce normal paraffins.

The high isomerization catalyst in the first isomerization unit 60 may include chlorided alumina, sulfated zirconia, tungstated zirconia or zeolite-containing isomerization catalysts. The high isomerization catalyst may be amorphous, e.g., based upon amorphous alumina, or zeolitic. A zeolitic catalyst would still normally contain an amorphous binder. The catalyst may comprise a sulfated zirconia and platinum as described in US 5,036,035 and EP 0666109 A1 or a platinum group metal on chlorided alumina as described in US 5,705,730 and US 6,214,764. Another suitable catalyst is described in US 5,922,639. US 6,818,589 discloses a catalyst comprising a tungstated support of an oxide or hydroxide of a Group IVB (IUPAC 4) metal, preferably zirconium oxide or hydroxide, at least a first component which is a lanthanide element and/or yttrium component, and at least a second component being a platinum-group metal component. An advantage of a non-chlorided catalyst, such as a sulfated zirconia catalyst, is the absence of chloride omitting further treatment of the effluent streams from the first isomerization unit 60. If chlorided alumina catalyst is used as the isomerization catalyst, a chloriding agent in line 63 will be added to the first isomerization feed stream 61.

The high isomerization process conditions in the first isomerization reactors 67, 68 and 69 include an average reactor temperature usually ranging from about 40° to about 250°C. High isomerization reactor operating pressures generally range from 1 MPa (145 psia) to about 5.5 MPa (800 psia) (g). Liquid hourly space velocities (LHSV) range from about 0.2 to about 25 volumes of hydrocarbon feed per hour per volume of catalyst. Hydrogen is admixed with or remains with the first isomerization feed to the first isomerization reactors 67-69 to provide a mole ratio of hydrogen to hydrocarbon feed that is greater than in a second isomerization unit 80. The hydrogen to hydrocarbon feed in the high isomerization conditions is from about 0.3 to 2 preferably about 0.5 to about 1.5 and a hydrogen partial pressure of about 1.0 MPa (150 psia) to about 2.4 MPa (350 psia) at the outlet of each reactor in the first isomerization unit 60. Higher hydrogen partial pressure in the first isomerization unit 60 is required to maintain stability of the high isomerization catalyst, to saturate and open aromatic and naphthenic rings, to promote hydrocracking reactions and to achieve high conversion to normal paraffins.

Contacting within the first isomerization unit 60 may be effected using the high isomerization catalyst in a fixed-bed system, a moving-bed system, a fluidized-bed system, or in a batch-type operation. The reactants may be contacted with the bed of high isomerization catalyst particles in upward, downward, or radial-flow fashion. The reactants may be in the liquid phase or in a mixed liquid-vapor phase when contacted with the high isomerization catalyst particles, with a mixed phase or vapor phase being preferred. The first isomerization unit 60 may be in a single reactor 67 or in two or more separate isomerization reactors 67, 68, and 69 with suitable means therebetween to ensure that the desired isomerization temperature is maintained at the entrance to each reactor.

The reactions in the first isomerization unit 60 generate an exotherm across the reactors so the isomerization effluent streams need to be cooled between reactors. For example, a first isomerization effluent stream from a first isomerization reactor 67 may be heat exchanged with the first isomerization feed stream in the first isomerization feed line 61 comprising isopentane mixed with hydrogen to cool the first isomerization effluent stream and heat the first isomerization feed stream. Moreover, a second isomerization effluent stream from a second high isomerization reactor 68 may be heat exchanged with the first isomerization feed stream upstream of the heat exchange with the first isomerate steam to cool the first isomerization effluent stream and heat the first isomerization feed stream. Additionally, a third isomerization effluent stream from the third isomerization reactor 69 may be heat exchanged with the first isomerization feed stream upstream of the heat exchange with the second isomerization effluent stream to cool the third isomerization effluent stream and heat the first isomerization feed stream. The third isomerate effluent stream is taken as the first isomerization effluent stream in line 65. Since hydrocracking reactions are accompanied by hydrogenation reactions that are very exothermic, two to five high isomerization reactors in sequence enable improved control of individual reactor temperatures and partial catalyst replacement without a process shutdown. A first isomerate stream comprising an increased concentration of normal paraffins exits the last isomerization reactor 69 in the first isomerization unit 60 in a first isomerate line 65.

In the isomerization of isopentanes, the isomerization reactors 67-69 produce isobutanes which will require isomerization to produce normal butanes. We have found that lower hydrogen partial pressure is beneficial to conversion of isobutanes and selectivity to normal butanes. Hence, excess hydrogen should be removed from the first isomerate stream in line 65 by means of a separator 100 to improve the hydrocarbon partial pressure.

The first isomerate stream in line 65 may be separated in a separator 100 in an embodiment at the pressure of the first isomerization unit 60. The separator 100 separates the first isomerate stream into a vapor isomerate stream in an overhead line 102 extending from an overhead of the separator 100 and a liquid isomerate stream in a bottoms line 104 extending from a bottom of the separator. The vapor isomerate stream is rich in gases, particularly hydrogen, and C3- hydrocarbons; whereas, the liquid isomerate stream is rich in C4-C6 paraffins.

The liquid isomerate stream in line 104 is charged to a second isomerization unit 80 which may be operated under intermediate isomerization conditions due to the presence of C4-C6 paraffins. The liquid isomerate stream in the separator bottoms line 104 may be combined with an optional second hydrogen stream in an optional second hydrogen line 82, optionally an isobutane rich stream in a net deisobutanizer overhead line 135 and optionally a fresh isobutane stream in a fresh isobutane line 81 to provide a second isomerization feed stream in a second isomerization feed line 84. The fresh isobutane may be from any section of the refinery such as from a saturation gas plant or a hydrocracking unit. The second isomerization feed stream is heated by heat exchange with a second isomerate stream and isomerized in the second isomerization reactors 86, 88. In the second isomerization unit 80, the C4-C6 paraffins, in the presence of hydrogen present in the liquid isomerate stream in line 104 or provided by the hydrogen line 82 and an intermediate isomerization catalyst, are converted to equilibrium levels of normal C4-C6 paraffins.

In addition to iso-normal paraffin isomerization, the conversion of isoparaffins via disproportionation reactions can also occur. The isobutanes can react via disproportionation to form propane and a pentane. The isopentanes can also isomerize to equilibrium producing normal pentane. Thus, there is an increase in the overall yield of the normal paraffins to propane, normal butane, normal pentane and normal hexane in the second isomerization reactors 86, 88.

The intermediate isomerization catalyst in the second isomerization reactors 86, 88 may include chlorided alumina, sulfated zirconia, tungstated zirconia or zeolite-containing isomerization catalysts. The intermediate isomerization catalyst may be amorphous, e.g., based upon amorphous alumina, or zeolitic. A zeolitic catalyst would still normally contain an amorphous binder. The intermediate isomerization catalyst may comprise a sulfated zirconia and platinum as described in US 5,036,035 and EP 0666109 A1 or a platinum group metal on chlorided alumina as described in US 5,705,730 and US 6,214,764. Another suitable catalyst is described in US 5,922,639. US 6,818,589 discloses a catalyst comprising a tungstated support of an oxide or hydroxide of a Group IVB (IUPAC 4) metal, preferably zirconium oxide or hydroxide, at least a first component which is a lanthanide element and/or yttrium component, and at least a second component being a platinum-group metal component. An advantage of a non-chlorided catalyst, such as a sulfated zirconia catalyst, is the absence of chloride omitting further treatment of the effluent streams from the second isomerization reactors 86, 88. If chlorided alumina catalyst is used as the intermediate isomerization catalyst, a chloriding agent in line 83 may be added to the second isomerization feed stream in line 84.

The intermediate isomerization conditions in the second isomerization reactors 86, 88 include reactor temperatures ranging from about 40°C to about 250° C, preferably at reactor temperatures ranging from 90°C to 230°C. Intermediate isomerization reactor operating pressures generally range from about 1 MPa (145 psia) to about 5.5 MPa (800 psia) (g). Liquid space velocity ranges from about 0.2 to about 25 volumes of hydrocarbon feed per hour per volume of catalyst. Hydrogen is admixed with the second isomerization feed stream to the second isomerization unit 80 to provide a mole ratio of hydrogen to hydrocarbon feed of from about 0.3 to about 1.5 and preferably 0.5 to about 1 and a hydrogen partial pressure of about 1.0 MPa (150 psia) to about 2.4 MPa (350 psia) at the outlet of each reactor in the second isomerization unit 80 depending on the concentration of C4s in the feed. The ratio of hydrogen to hydrocarbon at the reactor inlet and/or the reactor outlet can be selected based on the concentration of C4's in the feed. At low hydrogen excess, once-through hydrogen may be used to avoid need of a recycle compressor for recycling hydrogen.

Contacting within the second isomerization reactors 86, 88 may be effected using the catalyst in a fixed-bed system, a moving-bed system, a fluidized-bed system, or in a batch-type operation. The reactants may be contacted with the bed of catalyst particles in upward, downward, or radial-flow fashion. The reactants may be in the liquid phase, a mixed liquid-vapor phase, or a vapor phase when contacted with the catalyst particles, with a mixed phase or vapor phase being preferred. The second isomerization unit 80 may be in a single reactor 86 or two or more separate second isomerization reactors 86 and 88 with suitable means therebetween to ensure that the desired isomerization temperature is maintained at the entrance to each reactor. Even though the main reaction in the second isomerization reactors 86, 88 is isomerization of isoparaffins to normal paraffins which is endothermic, sufficient exothermic hydrogenation reactions occur causing the temperatures across the reactors to increase. Consequently, the second isomerate stream from an upstream reactor 86 must be cooled before going to a downstream reactor 88. For example, a first isomerization outlet stream from a first isomerization reactor 86 may be cooled by heat exchange with the second isomerization feed stream in line 84 and fed to a second isomerization reactor 88. Moreover, a second isomerization outlet stream from the second isomerization reactor 88 may be heat exchanged with the second isomerization feed stream to cool the second isomerization outlet stream upstream of the heat exchange with the first isomerization outlet stream. Two or more reactors in sequence enable improved isomerization through control of individual reactor temperatures and partial catalyst replacement without a process shutdown. The second isomerization outlet stream from the last isomerization reactor 88 in the second isomerization unit 80 is taken as a second isomerate stream comprising an increased concentration of normal paraffins in a second isomerate line 92. The second isomerate stream in the second isomerate line 92 may be fed to a depropanizer column 70 in a fractionation section 90 via a depropanizer feed line 93.

In the fractionation section 90, the depropanizer column 70 separates the second isomerate stream in line 92 perhaps combined with an optional diversion stream 56 into a depropanizer overhead stream comprising propane and lighter gases and a depropanized bottoms stream comprising C4+ paraffins. In an embodiment, a depropanizer column 70 separates the second isomerate stream in line 92 and perhaps the diversion stream in line 56 into a depropanizer overhead stream comprising propane and a depropanized bottoms stream comprising C4+ paraffins.

A depropanizer overhead stream is withdrawn from the depropanizer column 70 in a depropanizer overhead line 72 and condensed in a cooler and passed into a separator 74. A portion of the condensed depropanizer overhead stream is recycled to the depropanizer column 70 as reflux through a reflux line and the remaining portion of the condensed propane rich stream, is withdrawn in a net depropanizer overhead line 76. The depropanizer overhead stream in the line 76 may be charged to the steam cracking unit 150 or to a paraffin dehydrogenation process (not shown) perhaps after separation of lighter components from the propane. A depropanizer off gas stream comprising C2- hydrocarbons and light gases is taken from the separator overhead in a depropanizer off gas line 73. The depropanizer off gas in the off-gas line 73 may be scrubbed (not shown) to remove chlorine if a chloride isomerization catalyst is in the first isomerization unit 60 or the second isomerization unit 80 and passed to fuel gas processing or sent to further processing for further recovery of hydrogen and/or ethane which can be used as steam cracking feed to the steam cracking unit 150.

The depropanized bottoms stream is withdrawn from the depropanizer column 70 through a bottoms line 78 from which a portion of the depropanized bottoms stream flows through a reboiler line 77, a reboiler heater and returns to the depropanizer column 70. The remaining portion of the depropanized bottoms flows through a net depropanized bottoms line 79 rich in C4-C7 normal and iso-paraffins. The depropanizer column 70 operates in bottoms temperature range of about 90 to about 150°C and an overhead pressure range of about 1.3 to about 2.7 MPa and preferably about 1.7 to about 2.5 MPa.

In an embodiment, the remaining portion of the depropanized bottoms stream in the net depropanized bottoms line 79 rich in C4-C7 paraffins characterized as a C4+ paraffin stream is fed to a deisobutanizer column 130 to separate the C4+ stream into an iso-C4 paraffin rich overhead stream in an overhead line 132 and a normal C4 and C5-C7 paraffin rich bottoms stream in a bottoms line 134. The isobutane-rich, deisobutanizer overhead stream is withdrawn from the deisobutanizer column 130 in a deisobutanizer overhead line 132 and fully condensed in a cooler and passed into a separator 136. A portion of the condensed deisobutanizer overhead stream is recycled to the deisobutanizer column 130 as reflux through a reflux line and the remaining condensed deisobutanizer overhead stream is taken as the net deisobutanizer overhead stream rich in isobutane stream in the net deisobutanizer overhead line 135. The net deisobutanizer overhead stream may be fed to the second isomerization unit 80 to increase the concentration of normal butane in the isobutane stream in the deisobutanizer net overhead line 135. In an aspect, the net deisobutanizer overhead stream is recycled to the second isomerization unit 80 to isomerize isobutanes therein to normal butanes.

The deisobutanized bottoms stream is withdrawn from the deisobutanizer column 130 through a bottoms line 134 from which a portion of the deisobutanized bottoms stream flows through a reboiler line 137, a reboiler heater and returns to the deisobutanizer column 130. The remaining portion of the deisobutanized bottoms stream flows through a net deisobutanized bottoms line 139 which is rich in normal butane and heavier C5-C7 paraffins. Thus, the net deisobutanized bottoms stream comprising normal butane and C5-C7 paraffins may be recycled to the adsorbent separation vessel 12 in line 139 after heat exchange with the second isomerate stream in line 92. The deisobutanizer column 130 operates in bottoms temperature range of about 50 to about 100°C and an overhead pressure range of about 400 to about 800 kPa (gauge). Alternatively, the net deisobutanized bottoms stream comprising normal butane and C5-C7 paraffin in line 139 may be charged to the steam cracking unit 150.

The separator overhead line 102 may transport the first vapor isomerate stream to a hydrogen recovery unit 50 to provide a hydrogen rich stream in line 52 and a paraffin rich stream in line 54. The paraffin rich stream in line 54 comprises C4-C6 paraffins and may be charged to the second isomerization unit 80 in line 84. The hydrogen recovery unit 50 may be a pressure swing adsorption unit or a membrane unit. Up to 20 mol% of C1-C3 hydrocarbons may be fed to the second isomerization unit 80. If the concentration of C1-C3 hydrocarbons in the paraffin rich stream in line 54 is over the specification for the second isomerization unit 80, some of the paraffin rich stream can be diverted from line 54 in a diversion line 56 and fed to a fractionation section 90 in line 92.

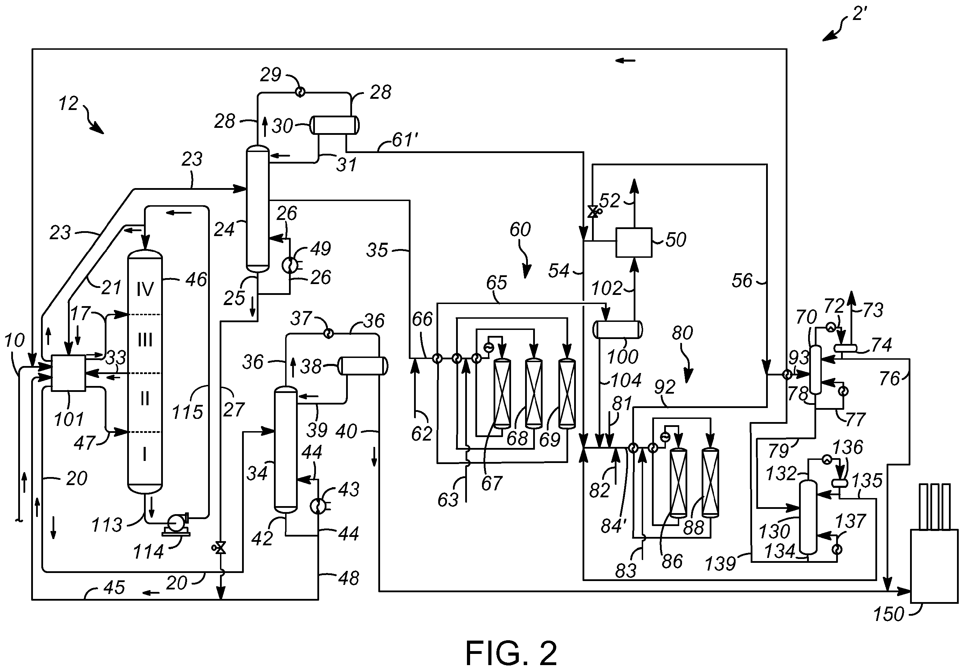

FIG. 2 shows an embodiment of a process and apparatus 2' in which butanes are present in the feed stream in line 10. Therefore, isobutanes are concentrated in the raffinate stream 23 and are separated in the raffinate column 24. Elements in FIG. 2 with the same configuration as in FIG. 1 will have the same reference numeral as in FIG. 1. Elements in FIG. 2 which have a different configuration as the corresponding element in FIG. 1 will have the same reference numeral but designated with a prime symbol ('). The configuration and operation of the embodiment of FIG. 2 is essentially the same as in FIG. 1 with the following exceptions.

In the alternative embodiment of FIG. 2, the raffinate feed stream comprising isoparaffins in line 23 in split into three streams: a first paraffin stream in a side line 35 that is rich in C5 to C7 non-normal paraffins, a second paraffin stream in the net raffinate overhead line 61 rich in isobutanes and isopentanes and a desorbent stream in line 25 rich in C9+ paraffins. The first paraffin stream in the side line 35 is charged to the first isomerization unit 60 which is operated under high isomerization conditions due to the C5-C7 paraffins in the first paraffin stream to provide the first isomerate stream. The second paraffin stream in line 61' is charged to the second isomerization unit 80 along with the paraffin rich stream in line 54, the liquid isomerate stream in line 104, the net deisobutanizer overhead stream in a deisobutanizer net overhead line 135 rich in isobutane, optionally a second hydrogen stream in a second hydrogen line 82 and optionally a fresh isobutane stream in a fresh isobutane line 81 to provide an intermediate isomerization feed stream in an intermediate isomerization feed line 84'. The second isomerization unit 80 is operated under intermediate isomerization conditions due to the presence of C4 and C5 paraffins in the second isomerization feed in line 84'. The remainder of the embodiment of FIG. 2 is configured and operated as described for FIG. 1.

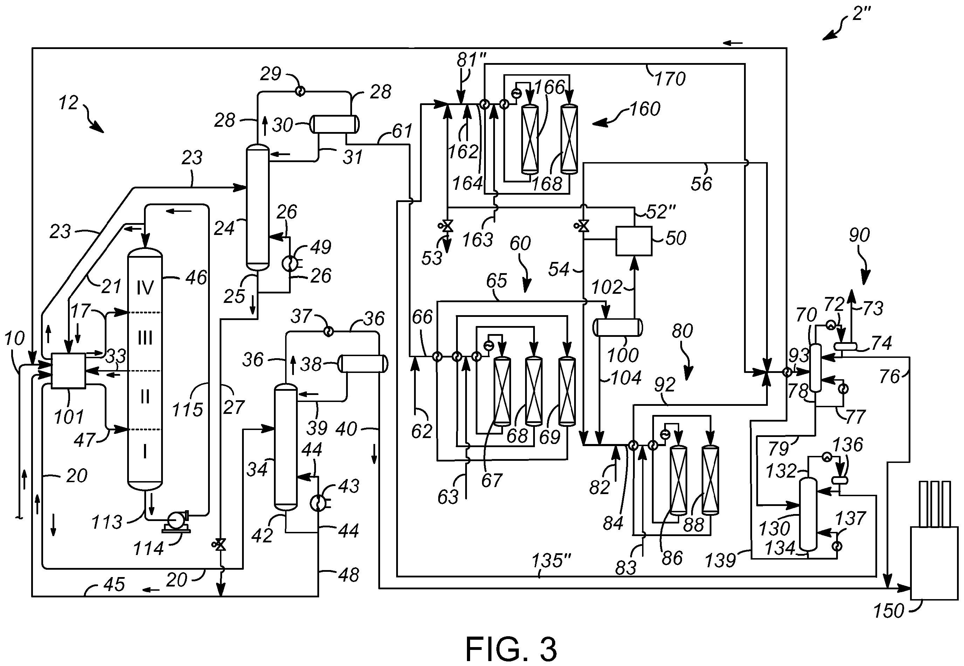

FIG. 3 shows an embodiment of a process and apparatus 2" in which an isobutane rich, net deisobutanizer overhead stream in the net deisobutanizer overhead line 135" from a deisobutanizer column 130 is isomerized in a third isomerization unit 160 to provide a third isomerate stream instead of in the second isomerization unit 80. Elements in FIG. 3 with the same configuration as in FIG. 1 will have the same reference numeral as in FIG. 1. Elements in FIG. 3 which have a different configuration as the corresponding element in FIG. 1 will have the same reference numeral but designated with a double prime symbol ("). The configuration and operation of the embodiment of FIG. 3 is essentially the same as in FIG. 1 with the following exceptions.

The net deisobutanizer overhead stream in the net deisobutanizer overhead line 135" rich in isobutane may be mixed with the hydrogen rich stream in line 52" and optionally a third hydrogen stream in a third hydrogen line 162 and/or a fresh isobutane stream in a fresh isobutane line 81" to provide a low isomerization feed stream in line 164. The fresh isobutane line 81" replaces the fresh isobutane line 81 to the second isomerization unit 80 in this embodiment. The hydrogen rich stream in line 52" may be purged in line 53 through a control valve thereon to ensure that the hydrogen partial pressure in the third isomerization unit 160 is sufficiently low.

The low isomerization feed stream in line 164 is charged to the third isomerization unit 160 which may comprise third isomerization reactors 166 and 168. The third isomerization unit 160 is operated under low isomerization conditions due to the feed being substantially or predominantly isobutane. The third isomerization feed stream is heated by heat exchange with a third isomerate stream and isomerized in the third isomerization reactors 166, 168 in the third isomerization unit 160. In the third isomerization reactors 166, 168, the isobutanes in the presence of hydrogen provided by the hydrogen rich stream in line 52" and a low isomerization catalyst, are converted into normal butanes to attain equilibrium levels.

The low isomerization catalyst in the third isomerization unit 160 may include chlorided alumina, sulfated zirconia, tungstated zirconia or zeolite-containing isomerization catalysts. The low isomerization catalyst may be amorphous, e.g., based upon amorphous alumina, or zeolitic. A zeolitic catalyst would still normally contain an amorphous binder. The low isomerization catalyst may comprise a sulfated zirconia and platinum as described in US 5,036,035 and EP 0666109 A1 or a platinum group metal on chlorided alumina as described in US 5,705,730 and US 6,214,764. Another suitable catalyst is described in US 5,922,639. US 6,818,589 discloses a catalyst comprising a tungstated support of an oxide or hydroxide of a Group IVB (IUPAC 4) metal, preferably zirconium oxide or hydroxide, at least a first component which is a lanthanide element and/or yttrium component, and at least a second component being a platinum-group metal component. An advantage of a non-chlorided catalyst, such as a sulfated zirconia catalyst, is the absence of chloride omitting further treatment of the effluent streams from the third isomerization unit 160. If chlorided alumina catalyst is used as the butane isomerization catalyst, a chloriding agent in line 163 will be added to the low isomerization feed stream 164.

The low isomerization conditions in the third isomerization unit 160 include reactor temperatures ranging from about 40°C to about 250° C, preferably at reactor temperatures ranging from about 90°C to about 230°C. Low isomerization reactor operating pressures generally range from about 690 kPa (100 psig) to about 4.2 MPa (600 psig) (gauge). Liquid space velocity ranges from about 0.2 to about 25 volumes of hydrocarbon feed per hour per volume of catalyst. Hydrogen is admixed with the third isomerization feed stream to the third isomerization unit 160 to provide a mole ratio of hydrogen to hydrocarbon of from about 0.01 to about 0.5, and suitably about 0.03 to about 0.4 and preferably about 0.05 to about 0.3 and a hydrogen partial pressure of about 69 kPa (10 psia) to about 1 MPa (150 psia) at the outlet of each reactor at low isomerization conditions. At low hydrogen excess, once through hydrogen may be used to avoid need of a recycle compressor for recycling hydrogen.

Up to 20 mol% of C1-C3 hydrocarbons may be fed to the third isomerization reactors 166, 168. If the concentration of C1-C3 hydrocarbons in the hydrogen rich stream in line 52" is over the specification for the low isomerization conditions in the third isomerization reactors 66, 68, some of the hydrogen rich stream in line 52" can be purged in line 53 through the control valve thereon. Make-up hydrogen can be provided in line 162 to off-set hydrogen purged to remove C1-C3 hydrocarbons from the feed stream to meet specifications if necessary.

Contacting within the third isomerization reactors 166, 168 may be effected using the catalyst in a fixed-bed system, a moving-bed system, a fluidized-bed system, or in a batch-type operation. The reactants may be contacted with the bed of catalyst particles in upward, downward, or radial-flow fashion. The reactants may preferably be completely in vapor phase. The third isomerization unit 160 may be in a single reactor 166 or two or more separate reactors 166 and 168 with suitable means therebetween to ensure that the desired low isomerization temperature is maintained at the entrance to each reactor.

Even though the main reaction in the third isomerization unit 160 is isomerization of isoparaffins to normal paraffins which is endothermic, sufficient exothermic hydrogenation reactions occur causing the temperatures across the reactors to increase. Consequently, the first isomerization discharge stream from an upstream reactor 166 must be cooled before going to a downstream reactor 168. For example, a first isomerization discharge stream from a first isomerization reactor 166 may be cooled by heat exchange with the third isomerization feed stream in line 164 and fed to a second isomerization reactor 168. Moreover, a second isomerization discharge stream from the second isomerization reactor 168 may be heat exchanged with the third isomerization feed stream comprising an isobutane-rich stream mixed with hydrogen to cool the second isomerization discharge stream upstream of the heat exchange with the first isomerization discharge stream. Two or more third isomerization reactors in sequence enable improved isomerization through control of individual reactor temperatures and partial catalyst replacement without a process shutdown. The second isomerization discharge stream is taken as a third isomerate stream comprising an increased concentration of normal butanes from the last reactor 168 in the third isomerization unit 160 in a third isomerate line 170. The third isomerate stream in line 170 may be fed to the depropanizer column 70 in the fractionation section 90. The remainder of the embodiment of FIG. 3 is configured and operates as described for FIG. 1.

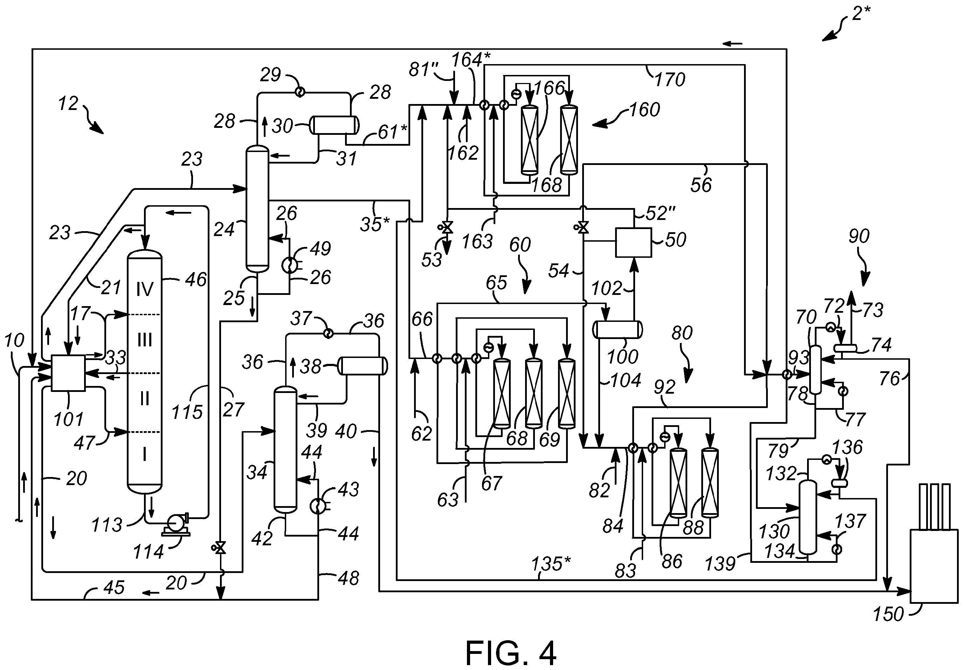

FIG. 4 shows an embodiment of a process and apparatus 2^{∗} in which a second paraffin stream comprising isobutane and isopentanes in the net raffinate overhead line 61^{∗} from the raffinate column 24 is isomerized in the third isomerization unit 160 with or without the deisobutanizer overhead stream 135^{∗} to provide the third isomerate stream. Elements in FIG. 4 with the same configuration as in FIGS. 2 and 3 will have the same reference numeral as in FIGS. 2 or 3, respectively. Elements in FIG. 4 which have a different configuration as the corresponding element in FIGS. 2 or 3, respectively, will have the same reference numeral but designated with a star symbol (^{∗}). The configuration and operation of the embodiment of FIG. 4 is essentially the same as in FIGS. 2 and 3 with the following exceptions.

The embodiment of FIG. 4 is applicable when the feed stream in line 10 to the adsorption separation unit 12 comprises greater than about 5 to about 10 wt% isobutanes which enter into the raffinate column 24 in raffinate line 23 and are separated in the second paraffin stream in the net raffinate overhead line 61^{∗} which is charged to the third isomerization unit 160. The third isomerization unit 160 is operated to isomerize a predominant concentration of isobutanes and is therefore operated at low isomerization conditions. The second paraffin stream in the net raffinate overhead line 61^{∗}, the hydrogen rich stream in line 52" and optionally a third hydrogen stream in a third hydrogen line 162 and/or a fresh isobutane stream in a fresh isobutane line 81" provide a third isomerization feed stream in line 164^{∗} and are charged to the third isomerization unit 160. The net deisobutanizer overhead stream in the net deisobutanizer overhead line 135 rich in isobutane may also be added to the third isomerization feed stream in line 164^{∗} and isomerized in the third isomerization unit 160 to provide a third isomerate stream in line 170. The third isomerization unit 160 is configured and operated to isomerize a predominantly isobutane feed to normal butanes in a third isomerate stream in line 170 and described for FIG. 3.

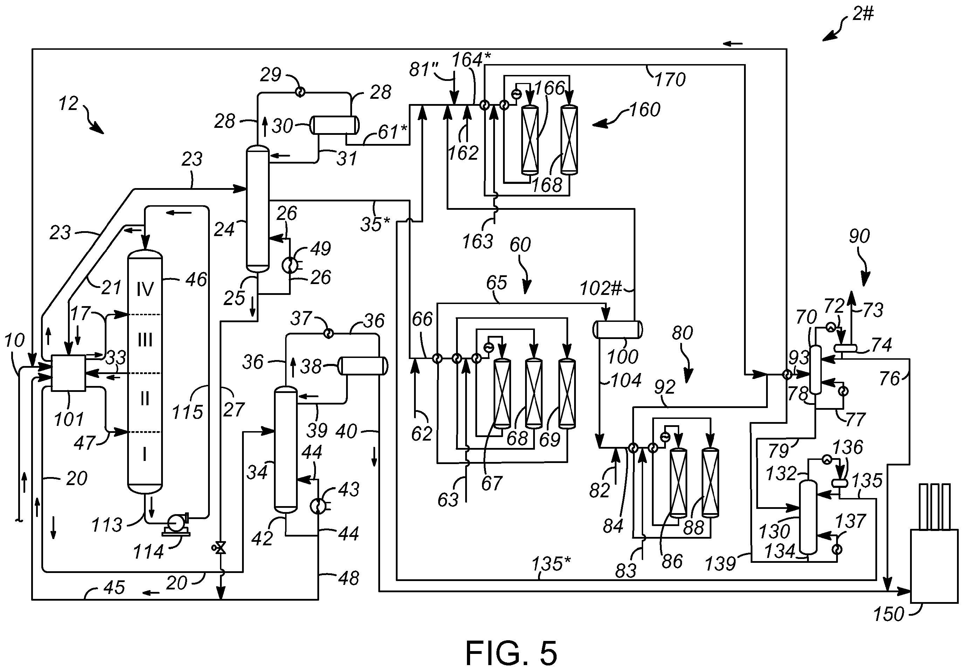

FIG. 5 shows an embodiment of a process and apparatus 2# in which the vapor isomerate stream in line 102# from the separator 100 is fed directly to the third isomerization unit 160 and isomerized in the third isomerization unit to provide the third isomerate stream with or without the net deisobutanizer overhead stream in the net deisobutanizer overhead line 135^{∗} and the second paraffin stream in line 61^{∗}. Elements in FIG. 5 with the same configuration as in FIG. 4 will have the same reference numeral as in FIG. 4. Elements in FIG. 5 which have a different configuration as the corresponding element in FIG. 4 will have the same reference numeral but designated with a hashtag symbol (#). The configuration and operation of the embodiment of FIG. 5 is essentially the same as in FIG. 4 with the following exceptions.

FIG. 5 omits the hydrogen recovery unit 50, so that the vapor isomerate stream in the separator overhead line 102# is fed directly, perhaps in its entirety, to the third isomerization unit 160. The vapor isomerate stream in the separator overhead line 102#, optionally a third hydrogen stream in a third hydrogen line 162 and/or a fresh isobutane stream in a fresh isobutane line 81" are charged to the third isomerization unit 160 and isomerized to provide a third isomerization feed stream in line 164^{∗}. The second paraffin stream in the net raffinate overhead line 61^{∗} and/or the net deisobutanizer overhead stream in the net deisobutanizer overhead line 135^{∗} rich in isobutane may also be added to the third isomerization feed stream in line 164^{∗} and isomerized in the third isomerization unit 160 to provide a third isomerate stream in line 170. The third isomerization unit 160 is configured and operated to isomerize a predominant isobutane feed to normal butanes in a third isomerate stream in line 170 at low isomerization conditions as described for FIG. 4.

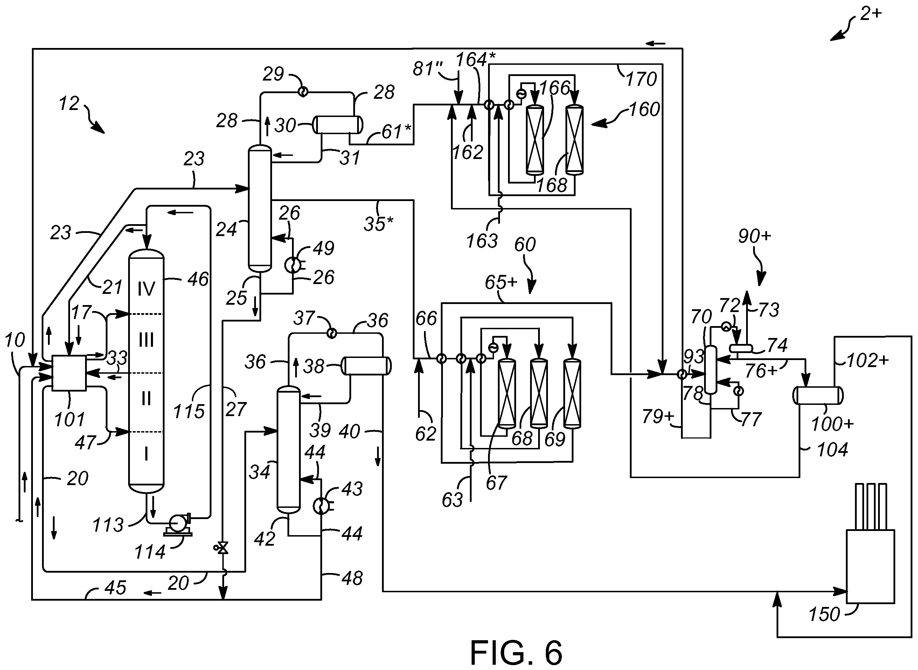

FIG. 6 shows an embodiment of a process and apparatus 2+ in which C4+ paraffins are separated from a first isomerate stream before separation of the vapor isomerate stream from the liquid isomerate stream in the separator 100+, to enable omission of the deisobutanizer column 130. Elements in FIG. 6 with the same configuration as in FIG. 5 will have the same reference numeral as in FIG. 5. Elements in FIG. 6 which have a different configuration as the corresponding element in FIG. 5 will have the same reference numeral but designated with a cross symbol (+). The configuration and operation of the embodiment of FIG. 6 is essentially the same as in FIG. 5 with the following exceptions.

In FIG. 6, the first isomerate stream in line 65+ from the first isomerization unit 60 comprising isomerized paraffins is depropanized in the depropanizer column 70 in a fractionation section 90+ that includes no deisobutanizer column 130. The depropanized bottoms stream in line 79+ rich in C4+ paraffins separated from the first isomerate stream is transported to the feed stream 10 to the adsorption separation unit 12 after heat exchange with the first isomerate stream in line 65+. The net depropanizer overhead stream in line 76+ is fed to the separator 100+ to remove excess hydrogen. The vapor isomerate stream in the overhead line 102+ rich in hydrogen and comprising C3- hydrocarbons can be further processed to recover hydrogen or sent to the steam cracking unit 150. The liquid isomerate stream in the bottoms line 104+ comprising C4 paraffins can be charged to the second isomerization unit 160 in a low isomerization feed stream in line 164^{∗}. In this embodiment the second isomerization reactors 166, 168 in the second isomerization unit 160 are so designated because only two isomerization units are employed. However, the second isomerization unit 160 is operated at low isomerization conditions because it processes substantially or predominantly isobutane feed. The remainder of the operation and configuration of the embodiment of FIG. 6 is just as is described for FIG. 5.

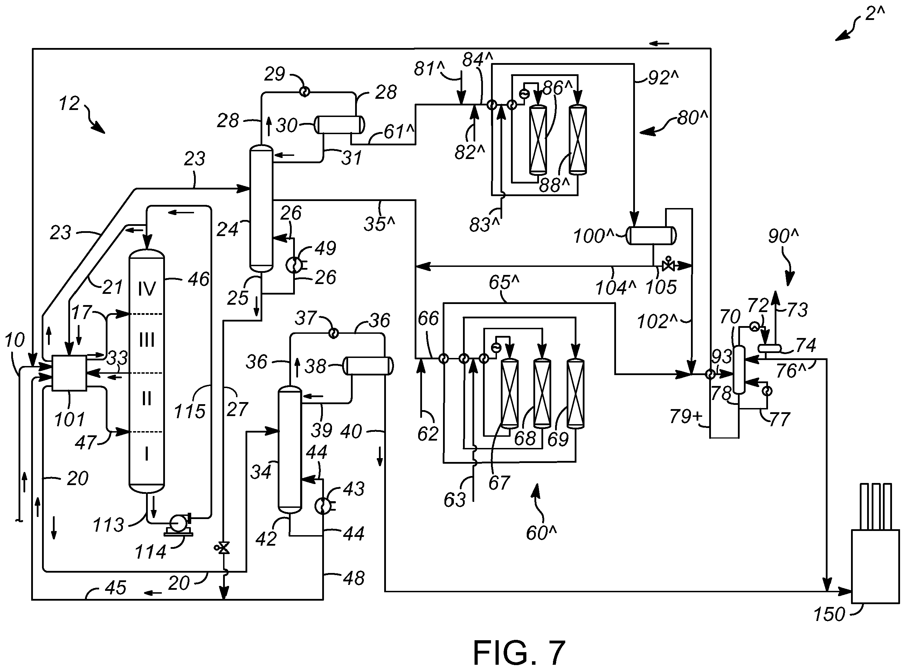

FIG. 7 depicts an embodiment of a process and apparatus 2^ in which the second paraffin stream is isomerized with the liquid isomerate stream to provide a second isomerate stream. However, the raffinate net overhead stream in line 61^ is the first paraffin stream, and the raffinate side stream in the side line 35^ is the second paraffin stream unlike in previous embodiments. Elements in FIG. 7 with the same configuration as in FIG. 6 will have the same reference numeral as in FIG. 6. Elements in FIG. 7 which have a different configuration as the corresponding element in FIG. 6 will have the same reference numeral but designated with a carat symbol (^). The configuration and operation of the embodiment of FIG. 7 is essentially the same as in FIG. 6 with the following exceptions.

The first paraffin stream in the net raffinate overhead line 61^ comprising C4 and C5 isoparaffins is isomerized in a first isomerization unit 80^ with a first hydrogen stream in line 82^ and an optional a fresh isobutane stream in line 81^ and/or a chloriding agent in line 83^ to produce a first isomerate stream in line 92^ operated at intermediate isomerization conditions as described for the second isomerization unit 80 in FIG. 1. The first isomerate stream in line 92^ is separated in a separator 100^ without pressure reduction to provide a vapor isomerate stream in line 102^ and a liquid isomerate stream in line 104^. The liquid isomerate stream in line 104^ is mixed with the first paraffin stream in the side line 35^ and are together isomerized in second isomerization reactors 67-69 in a second isomerization unit 60^ operated at high isomerization conditions as described for the first isomerization unit in FIG. 1 to produce a second isomerate stream in line 65^. The liquid isomerate stream in the second isomerization reactors 67-69 provides additional material to control the exotherm. A portion of the liquid isomerate stream in line 104^ in line 105 through a control valve thereon may be mixed with the vapor isomerate stream in line 102^. The vapor isomerate stream in line 102^ may be mixed with the second isomerate stream in line 65^ and depropanized in the depropanizer 70 in the fractionation section 90^. The net depropanizer overhead stream in line 76^ may be fed to the steam cracking unit. Like the embodiment of FIG. 6, the fractionation section 90^ omits a deisobutanizer column.

The process and apparatus separate isomerization of C4 paraffins from isomerization of C5 paraffins because advantageous conditions are different for each isomerization reactant. Conversion and selectivity to normal paraffins is increased by reducing the hydrogen to hydrocarbon ratio for paraffin feeds with substantial butanes. The separator is used to remove excess hydrogen from a first isomerate comprising butanes before a second isomerization step of the additional butanes.

### SPECIFIC EMBODIMENTS

While the following is described in conjunction with specific embodiments, it will be understood that this description is intended to illustrate and not limit the scope of the preceding description and the appended claims.

A first embodiment of the disclosure is a process for isomerizing isoparaffins to normal paraffins comprising isomerizing a first paraffin stream to provide a first isomerate stream; separating the first isomerate stream into a vapor isomerate stream and a liquid isomerate stream; and isomerizing the first liquid isomerate stream to provide a second isomerate stream. An embodiment of the disclosure is one, any or all of prior embodiments in this paragraph up through the first embodiment in this paragraph further comprising separating the first vapor isomerate stream into a hydrogen rich stream and a paraffin rich stream. An embodiment of the disclosure is one, any or all of prior embodiments in this paragraph up through the first embodiment in this paragraph further comprising charging at least a portion of the paraffin rich stream to the second isomerizing step. An embodiment of the disclosure is one, any or all of prior embodiments in this paragraph up through the first embodiment in this paragraph further comprising steam cracking the hydrogen rich stream. An embodiment of the disclosure is one, any or all of prior embodiments in this paragraph up through the first embodiment in this paragraph further comprising separating a paraffin feed stream into the first paraffin stream and a second paraffin stream. An embodiment of the disclosure is one, any or all of prior embodiments in this paragraph up through the first embodiment in this paragraph further comprising isomerizing the second paraffin stream with the first liquid isomerate stream to provide the second isomerate stream. An embodiment of the disclosure is one, any or all of prior embodiments in this paragraph up through the first embodiment in this paragraph further comprising fractionating the second isomerate stream to provide a normal butane stream and an isobutane stream; and isomerizing the isobutane stream optionally with the hydrogen rich stream to provide a third isomerate stream. An embodiment of the disclosure is one, any or all of prior embodiments in this paragraph up through the first embodiment in this paragraph further comprising isomerizing the second paraffin stream to provide a third isomerate stream. An embodiment of the disclosure is one, any or all of prior embodiments in this paragraph up through the first embodiment in this paragraph further comprising separating the first vapor isomerate stream into a hydrogen rich stream and a paraffin rich stream and charging the hydrogen rich stream to the step of isomerizing the second paraffin stream to provide a third isomerate stream. An embodiment of the disclosure is one, any or all of prior embodiments in this paragraph up through the first embodiment in this paragraph further comprising isomerizing the paraffin rich stream with the liquid isomerate stream to provide the second isomerate stream. An embodiment of the disclosure is one, any or all of prior embodiments in this paragraph up through the first embodiment in this paragraph further comprising isomerizing the vapor isomerate stream and the second paraffin stream to provide a third isomerate stream. An embodiment of the disclosure is one, any or all of prior embodiments in this paragraph up through the first embodiment in this paragraph further comprising separating C4+ paraffins from the first isomerate stream before the separation step and isomerizing the first liquid isomerate stream to provide the second isomerate stream. An embodiment of the disclosure is one, any or all of prior embodiments in this paragraph up through the first embodiment in this paragraph further comprising separating a paraffin feed stream into the first paraffin stream and a second paraffin stream and isomerizing the liquid isomerate stream with the second paraffin stream to provide the second isomerate stream. An embodiment of the disclosure is one, any or all of prior embodiments in this paragraph up through the first embodiment in this paragraph further comprising fractionating the second isomerate stream to provide a normal butane stream and an isobutane stream; and isomerizing the isobutane stream. An embodiment of the disclosure is one, any or all of prior embodiments in this paragraph up through the first embodiment in this paragraph wherein the fractionating step includes depropanizing the second isomerate stream to provide a depropanizer overhead stream and a depropanized bottom stream and optionally steam cracking the depropanizer overhead stream and deisobutanizing the depropanized bottom stream to provide the normal butane stream and the isobutane stream. An embodiment of the disclosure is one, any or all of prior embodiments in this paragraph up through the first embodiment in this paragraph wherein the isobutane stream is isomerized in a third isomerization unit to provide a third isomerate stream. An embodiment of the disclosure is one, any or all of prior embodiments in this paragraph up through the first embodiment in this paragraph further comprising separating a paraffin feed stream into the first paraffin stream and a second paraffin stream and isomerizing the second paraffin stream with the liquid isomerate stream to provide the second isomerate stream. An embodiment of the disclosure is one, any or all of prior embodiments in this paragraph up through the first embodiment in this paragraph further comprising separating a paraffin feed stream into the first paraffin stream and a second paraffin stream and isomerizing the isobutane stream with the liquid isomerate stream to provide a third isomerate stream. An embodiment of the disclosure is one, any or all of prior embodiments in this paragraph up through the first embodiment in this paragraph further comprising isomerizing the second paraffin stream with the liquid isomerate stream to provide a second isomerate stream.

A second embodiment of the disclosure is a process for isomerizing isoparaffins to normal paraffins comprising isomerizing a C5 paraffin stream to provide a C5 isomerate stream; separating the C5 isomerate stream into a first vapor isomerate stream and a first liquid isomerate stream; and isomerizing the first liquid isomerate stream to provide a C4 isomerate stream.

Without further elaboration, it is believed that using the preceding description that one skilled in the art can utilize the present disclosure to its fullest extent and easily ascertain the essential characteristics of this disclosure, without departing from the spirit and scope thereof, to make various changes and modifications of the disclosure and to adapt it to various usages and conditions. The preceding preferred specific embodiments are, therefore, to be construed as merely illustrative, and not limiting the remainder of the disclosure in any way whatsoever, and that it is intended to cover various modifications and equivalent arrangements included within the scope of the appended claims.

In the foregoing, all temperatures are set forth in degrees Celsius and, all parts and percentages are by weight, unless otherwise indicated.

## Claims

1. A process for isomerizing isoparaffins to normal paraffins comprising:
isomerizing a first paraffin stream to provide a first isomerate stream;
separating said first isomerate stream into a vapor isomerate stream and a liquid isomerate stream; and
isomerizing said liquid isomerate stream to provide a second isomerate stream.

2. The process of claim 1 further comprising separating the first vapor isomerate stream into a hydrogen rich stream and a paraffin rich stream.

3. The process of claim 2 further comprising charging at least a portion of said paraffin rich stream to said second isomerizing step.

4. The process of claim 2 further comprising steam cracking the hydrogen rich stream.

5. The process of claim 1 further comprising separating a paraffin feed stream into said first paraffin stream and a second paraffin stream.

6. The process of claim 5 further comprising isomerizing said second paraffin stream with said liquid isomerate stream to provide said second isomerate stream.

7. The process of claim 2 further comprising fractionating said second isomerate stream to provide a normal butane stream and an isobutane stream; and isomerizing said isobutane stream optionally with said hydrogen rich stream to provide a third isomerate stream.

8. The process of claim 5 further comprising isomerizing said second paraffin stream to provide a third isomerate stream.

9. The process of claim 8 further comprising separating the first vapor isomerate stream into a hydrogen rich stream and a paraffin rich stream and charging said hydrogen rich stream to said step of isomerizing said second paraffin stream to provide a third isomerate stream.

10. The process of claim 9 further comprising isomerizing said paraffin rich stream with said liquid isomerate stream to provide said second isomerate stream.

11. The process of claim 10 further comprising isomerizing said vapor isomerate stream and said second paraffin stream to provide a third isomerate stream.

12. The process of claim 1 further comprising separating C4+ paraffins from said first isomerate stream before said separation step and isomerizing said liquid isomerate stream to provide said second isomerate stream.

13. The process of claim 12 further comprising separating a paraffin feed stream into said first paraffin stream and a second paraffin stream and isomerizing said liquid isomerate stream with said second paraffin stream to provide said second isomerate stream.

14. The process of claim 1 further comprising fractionating said second isomerate stream to provide a normal butane stream and an isobutane stream; and isomerizing said isobutane stream.

15. The process of claim 14 wherein said fractionating step includes depropanizing said second isomerate stream to provide a depropanizer overhead stream and a depropanized bottom stream and optionally steam cracking said depropanizer overhead stream and deisobutanizing said depropanized bottom stream to provide said normal butane stream and said isobutane stream.
